# EUROPEAN PATENT APPLICATION

(11) **EP 1 867 722 A1**
(43) Date of publication of application: **19.12.2007**
(21) Application number: 06076248.1
(22) Date of filing: 16.06.2006
(51) Int. Cl.: C12N 15/82, A01H 5/00

(54) **Synthesis of lewis X epitopes on proteins in plants**

(71) Applicant: Plant Research International B.V., 6708 PB Wageningen (NL)
(72) Inventor: Rouwendal, Gerard Johan Adolph, 6666 HM Heteren (NL); Bosch, Hendrik Jan, 6708 NA Wageningen (NL); Florack, Dionisus Elisabeth Antonius, 6708 MD Wageningen (NL)
(74) Representative: van Loon, C.J.J.

(57) **Abstract**

The invention comprises a method to produce a Lewis X epitope on N-linked glycans on glycoproteins in a plant, plant part or plant cells, comprising expression of a nucleotide sequence encoding a galactosyltransferase, which catalyzes the addition of galactose in β1,4-linkage to N-linked glycans, and expression of a nucleotide sequence encoding a fucosyltransferase, which catalyzes the addition of fucose in α1,3-linkage to the N-acetylglucosamine of Galβ1-4GlcNAc structures on N-linked glycans, and expression of one or more nucleotide sequences encoding one or more glycoproteins, which are able to be provided with an N-linked glycan, in said plant, plant part or plant cell. The fucosyltransferase comprises preferably the catalyticaal active domain of FUT9 from *T. nigroviridis.* Also part of the invention are hybrid enzymes with said domain and an N-terminal part which confers expression in the Golgi apparatus.

## Description

The invention relates to the field of plant genetic engineering, more specifically related to production of glycosylation patterns on (recombinantly produced) plant proteins.

Glycoproteins are proteins which have undergone so-called posttranslational modification, in the sense that sugar groups are covalently coupled to the protein. It was found that approximately 50% of the proteins produced by a living cell undergo glycosylation. Further, it appeared that the type of glycosylation differs among the eukaryotes, meaning that different types of sugar moieties are used and/or a different order of attaching the moieties to the protein backbone, thereby forming a variety of branching structures. Glycosylation of proteins is highly regulated and changes during differentiation, development, under different physiological-and cell culture-conditions and in disease. The glycosylation of recombinant proteins, especially those destined for potential administration to human subjects, is of critical importance, while it affects many properties of the (glyco)protein, such as proper folding, protease resistance/sensitivity, intracellular trafficking and compartmentalization, intermolecular affinities and tissue targeting. Glycosylation further profoundly affects biological half-life, activity, function, solubility, clearance from circulation, and crucial for intended pharmaceutical glycoproteins, antigenicity.

The cells of nonhuman species do not glycosylate their proteins in the same way as human cells do. In many cases, the differences are profound. Overall, the species most distant to humans in evolutionary terms, such as bacteria, yeasts, fungi, insects and plants-the species used most commonly in expression systems-have glycosylation repertoires least like our own (for a review see for example Spiro (2002), Glycobiology Vol. 12, no. 4, pp.43R-56R).

Many expression systems are nowadays employed or explored as biopharmaceutical production platforms (*E*. *coli,* CHO cells, insect cells, plants, algae, yeasts, filamentous fungi, etc.). Suitability is determined by a lot of parameters, like for example safety, efficacy, costs and not in the least by posttranslational modifications that are either required or that need to be absent.

Bacteria, as being mostly used for large scale recombinant protein production, have the disadvantage that they do not glycosylate the recombinantly expressed proteins.

As expected, glycosylation in mammalian cell expression systems most resembles human glycosylation. However, mammalian cell lines that are able to replicate human-like glycoprotein processing have several drawbacks including low protein titers, long fermentation times, heterogeneous products, and ongoing viral containment issues. For example, it appeared that CHO cells which are genetically engineered to produce large quantities of a specific protein often do not maintain the proper level of glycosylation. This results in low yields of usable product, which contributes to the cost and complexity of producing these glycoproteins.

Glycosylation in plants has been proposed as an alternative, but achieving the desired glycosylation patterns remains a problem with these systems and is a significant barrier to their widespread adoption for manufacturing proteins.

Of the different types of glycosylation, N-linked glycosylation is important, because e.g. monoclonal antibodies secreted by recombinant cell expression systems or transgenic organisms nearly always contain a single glycosylation site at Asn 297, a site located in the CH2 domain within the Fc region of the molecule. Monoclonal antibodies lacking the N-linked glycan in the CH2 domain exhibit normal antigen binding mediated by hypervariable regions of the Fab portions of the molecule, but are deficient in Fc effector functions such as antibody dependent cellular cytotoxicity (ADCC) and complement mediated lysis (CDL). These and other effector functions require molecular recognition between the Fc domain of the IgG molecule and proteins such as Fc receptors on the surfaces of immunocytes (ADCC) or soluble proteins that initiate the complement cascade (CML). Glycosylation is important to antibody effector function, such as ADCC and CML, that mediate killing of tumor cells by antibodies developed as oncolytics.

In plants, processing of N-linked glycans occurs along the transport pathway as the glycoproteins move from the endoplasmatic reticulum through the Golgi apparatus to their final destination. Glycosidases and glycosyltransferases located in the Golgi apparatus successively modify the oligosaccharide precursors to complex type N-glycans of limited sizes, containing β(1,2)-xylose and/or α(1,3)-fucose residues that are specific to plant glycoproteins. Recently, it has been established (Fichette-Lainé, A.-C. *et al.,* 1997, The Plant J. 12:14110-1417) plants are able to make larger mono- and bi-antennary N-linked complex glycans, which are composed of Lewis a (Le^{a}) antigens, comprising the carbohydrate sequence Galβ1-3[Fucα1-4]GlcNAc. However, Lewis x epitopes, which comprise the carbohydrate sequence Galβ1-4[Fuca1-4]GlcNAc, do not naturally occur in plants, since plants lack the enzymes for β1,4-galactosyltransferase (GalT) and α1,3-fucosyltransferase. It has, however, been demonstrated in our lab (Bakker, H., et al., 2001, Proc. Natl. Acad. Sci. USA 98:2899-2904; Bakker, H. et al., 2006, Proc. Natl. Acad. Sci. USA, 103:7577-7582) that GalT can be expressed in plants. It is deemed useful to develop Lewis X (Le^{X}) epitopes on such antennary N-linked glycans to render the galactose residue insensitive to β-galactosidases, enzymes that are very useful for sequential glycosidase sequencing of oligosaccharides (Kobata, A, 1979, Anal. Biochem. 100:1-14; Zeleny, R. et al., 1993, Anal. Biochem. 246:96-101).

Thus, one of the goals of the present invention was to develop a method for the production of Lewis X epitopes on N-linked glycans in plants.

### SUMMARY OF THE INVENTION

The invention comprises a method to produce a Lewis x epitope on N-linked glycans on glycoproteins in a plant, plant part or plant cells, comprising
a. expression of a nucleotide sequence encoding a galactosyltransferase, which catalyzes the addition of galactose in ß1,4-linkage to N-linked glycans, and
b. expression of a nucleotide sequence encoding a fucosyltransferase, which catalyzes the addition of fucose in α1,3-linkage to the N-acetylglucosamine of Galß1-4GlcNAc structures on N-linked glycans, and
c. expression of one or more nucleotide sequences encoding one or more glycoproteins, which are able to be provided with an N-linked glycan, in said plant, plant part or plant cell

Preferably in this method said nucleotide sequence encoding a galactosyltransferase is a nucleotide sequence encoding at least the catalytically active domain of human GalT, more in particular, said nucleotide sequence encoding a galactosyltransferase also comprises an N-terminal domain, which confers localisation to the Golgi apparatus, preferably wherein said N-terminal domain is the N-terminal domain of *Arabidopsis thaliana* xylosyltransferase.

Also preferably in this method said nucleotide sequence encoding a fucosyltransferase is a nucleotide sequence encoding at least the catalytically active domain of α1,3-fucosyltransferase, preferably FUT9, more preferably the FUT9 enzyme of *Tetraodon nigroviridis,* more in particular, said nucleotide sequence encoding a fucosyltransferase also comprises an N-terminal domain, which confers localisation to the Golgi apparatus, preferably wherein said N-terminal domain is the N-terminal domain of *Arabidopsis thaliana* xylosyltransferase.

Another embodiment of the invention is a method to render a galactose residue on N-linked glycans in plant glycoproteins insensitive to galactosidase by incorporating it within a Lewis X epitope according to a method according to the invention.

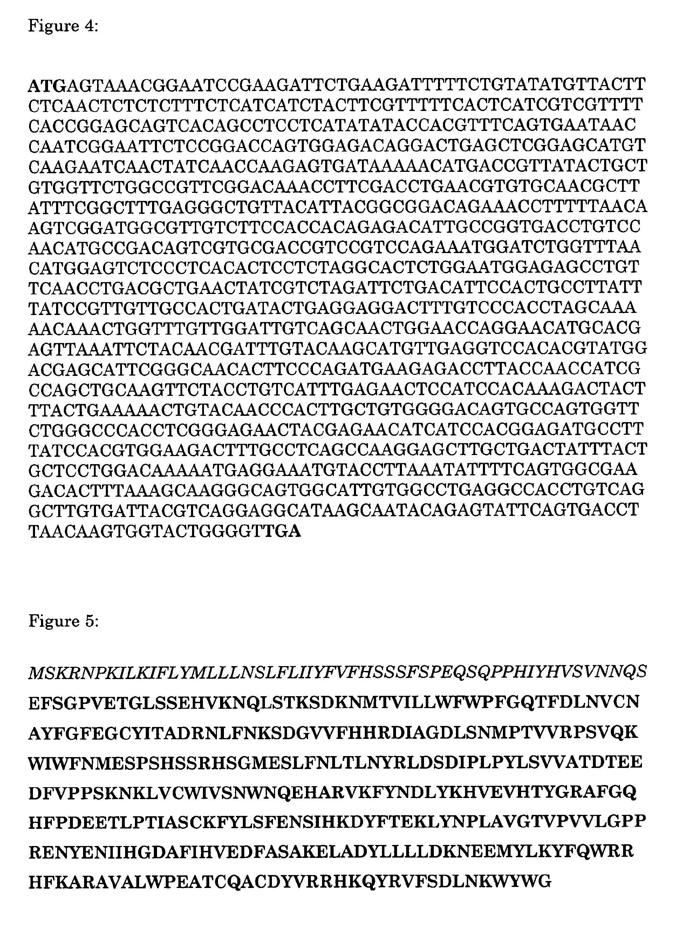

Still another embodiment of the invention is a hybrid α1,3-fucosyltransferase, comprising an N-terminal domain, which confers localisation to the Golgi apparatus, preferably wherein said N-terminal domain is the N-terminal domain of *Arabidopsis thaliana* xylosyltransferase, and the catalytic domain of a FUT9, preferably T. *nigroviridis* FUT9, said hybrid α1,3-fucosyltransferase preferably having the amino acid sequence from Fig. 5

Also encompassed in the present invention is a transgenic plant comprising a nucleotide sequence encoding a ß1,4-galactosyltransferase and a nucleotide sequence encoding an α1,3-fucosyltransferase (FUT9), preferably a transgenic plant capable of producing Lewis X epitopes on N-linked glycans of glycoproteins.

### LEGENDS TO THE FIGURES

Fig. 1. MALDI-TOF MS spectra of sodiated N-glycans from xFxG3 (panel A) and xFxG4 (panel B).
Fig. 2. MALDI-TOF MS spectra of sodiated N-glycans from xylGalT12 (panel A) and xFxG1 (panels B and C). The samples in panels B and C were obtained following release by PNGase A and F, respectively.
Fig. 3. MALDI-TOF MS spectra of sodiated N-glycans from xFxG1 treated with ß1,4-galactosidase (panel B), α1-3,4-fucosidase (panel C), stepwise with α1-3,4-fucosidase and ß1,4-galactosidase (panel D) and untreated control (panel A).
Fig. 4 Coding DNA sequence for the hybrid xylFUT9 enzyme. The start and stop codons have been indicated in bold capitals.
Fig. 5. Amino acid sequence of the hybrid xylFUT9 enzyme. The sequence derived from the *A. thaliana* xylosyltransferase is indicated in italics and the sequence derived from the *T. nigroviridis* FUT9 is in bold capitals. The not bold or italics E residue is (part of) the linker connecting the N-terminal domain and the catalytic domain

### DETAILED DESCRIPTION OF THE INVENTION

Lewis epitopes, which can occur on glycoproteins and glycolipids, have first been discovered in blood group typing, where the Lewis determinants are structurally related to determinants of the ABO and the H/h blood group systems. In humans, the Lewis epitopes are the product of the interaction of two different fucosyltransferases, FUT2, which encodes α1,2-fucosyltransferase and FUT3, which encodes α1,3/4-fucosyltransferase. Depending on the configuration of the substrate Le a and b epitopes are formed or Lewis X and Y epitopes. Le^{x} is a bi-antennary glycan, comprising a GlcNac moiety, which is ß1,2-linked to the mannosylated chitobiose core of the N-glycan, to which a galactose moiety is linked through a ß1,4 linkage and a fucose moiety is linked through an α1,3 linkage. In humans, the epitope is frequently sialylated.

Le^{x}, like the other Lewis determinants, probably plays a role in protein-membrane interactions, like recognition and adhesion processes. In mice, it is a marker of embryonic stem cells, embryonal carcinoma cells and multipotential cells of early embryos. Le^{x} is not expressed in human embryonic stem cells, embryonal carcinoma cells or inner cell mass cells, but it is a marker of primordial germ cells or multipotential stem cells derived from primordial germ cells. Also, both in mice and humans, Le^{X} is expressed in neural stem cells.

N-glycosylation of a monoclonal antibody (mAb) may have a major impact on its therapeutic merits. Bakker *et al.* (2006) demonstrate that expression of a hybrid enzyme (called xylGalT), consisting of the N-terminal domain of *Arabidopsis thaliana* xylosyltransferase and the catalytic domain of human β-1,4-galactosyltransferase I (GalT), in tobacco causes a sharp reduction of N-glycans with potentially immunogenic core-bound xylose (Xyl) and fucose (Fuc) residues as shown by Western blot and MALDI-TOF MS analysis. A radioallergosorbent test inhibition assay with proteins purified from leaves of WT and these transgenic tobacco plants using sera from allergic patients suggested a significant reduction of potential immunogenicity of xylGalT proteins. A mAb purified from leaves of plants expressing xylGalT displayed an N-glycan profile that featured high levels of galactose, undetectable xylose, and a trace of fucose. Hence, a transgenic plant expressing the hybrid GalT, wherein the enzymatic function by using the N-terminal domain of the xylosyltransferase is localised in the Golgi apparatus, might yield more effective and safer monoclonals for therapeutic purposes than WT plants and even transgenic plants expressing the unchanged GalT. However, it is envisaged that in stead of the N-terminal domain of xylosyltransferase, any sequence, which is capable of localising the expression of the protein to the Golgi apparatus, would be useful for making a hybrid GalT enzyme as used in the present invention. Many different types of N-terminal domains from different origins (plant, animal, fungal) are suitable as long as they confer Golgi-localization onto the catalytic domain. For example, the N-terminal domains of many plant and animal glycosyltransferases may be suitable

Protection of the galactose moiety on the above mentioned monoclonal antibodies is preferably done according to the invention by introducing a Lewis X epitope on the N-glycan through the action of an α1,3-fucosyltransferase.

Of the α1,3-fucosyltransferases, the mouse FUT9 gene was the first to be cloned and subsequently shown to be highly homologous to its human counterpart (Kudo, T., et al., 1998, J. Biol. Chem. 273:26729-26738; Kaneko, M. et al., 1999, FEBS Lett. 452:237-242). It proved to be a distant member of a family of at least 6 mammalian a1,3-fucosyltransferases, 5 of which were capable of fucosylating the nonreducing ends of glycans with type 2 chains (Nishihara, S. et al., 1999, FEBS Lett. 462:289-294). The mouse FUT9 transcript was the only one encoding an enzyme capable of synthesizing Fuc(α1-3)GlcNac linkages that was expressed in brain and kidney (Cornelli, E.M. et al., 2006, Glycobiology 16:117-131). Therefore, it was one of the (many) candidates that could be responsible for the synthesis of Le^{X} epitopes as encountered in these tissues. FUT9 displays a deviant acceptor specificity compared to the other 4 α1,3-FUTs as it preferentially fucosylates the distal GlcNac residue in a poly-LN chain. Nakayama, F. et al. (J. Biol. Chem. 276:16100-16106, 2001) showed that the expression of the LeX epitope in human mature granulocytes is directed by FUT9. CAECAM1, a surface glycoprotein on granulocytes, carries a large number of LeX epitopes (Lucka, L. et al., 2005, Glycobiol. 15:87-100), but FUT9-dependent fucosylation of CAECAM1 appeared to be rather specific as shown by transfection studies with the gene encoding this enzyme, which revealed no detectable transfer of fucose to other cellular proteins than the co-transfected CEACAM1 (Bogoevska, V. et al., 2006, Glycobiol. 16:197-209). Thus, although FUT9 showed the activity, nothing was known on its specificity, especially not when the enzyme would be exerting its function in a transgenic environment, especially in a plant cell.

The FUT9 homologue from pufferfish *(T. nigroviridis)* was 67% homologous at the amino acid level to the human FUT9. Although the human gene would also be useful in the present invention, the choice for this pufferfish gene source was predominantly determined by considerations regarding sequence characteristics, that would lead to optimal expression in plants. In this respect, the fish FUT9, with a G+C content of 58% was deemed superior to the human gene with a G+C content of 41% and a long stretch of more than 100 basepairs with a G+C level of below 30%. It would of course also be possible to change the coding sequence for the human FUT9 enzyme in such a way, that the G+C content increase while still coding for the same amino acid sequence. A person skilled in the art will know that more nucleotide triplets will code for the same amino acid and will be able to choose a DNA sequence coding for the same enzyme with an increased G+C content. In this way any other nucleotide sequence coding for an enzyme providing a functional FUT9 catalytic domain may be used and/or adapted for optimal expression in plants.

Although the fish FUT9 enzyme could have been used as such, the choice to make a hybrid enzyme was imposed by the use of the hybrid GalT, which was chosen not only for its ability to induce high-level galactosylation, but also for the reason that this is associated with the occurrence of readily detectable N-glycan GlcNacMan₅GlanNac₂ that is presumably due to de-galactosylation of the main galactosylated N-glycan, (Bakker *et al.,* 2006). But, as the xylGalT hybrid enzyme tends to be localized in the medial-Golgi, it had to be ensured that the FUT9 activity would also be present in this compartment. Therefore, in our experiments the FUT9 catalytic domain was fused with the same CTS-region as the GalT catalytic domain, thereby providing the presence of this enzyme in the neighborhood of the GalT enzyme. However, it is envisaged that in stead of the N-terminal domain of *Arabidopsis thaliana* xylosyltransferase, any sequence, which is capable of localising the expression of the protein to the Golgi apparatus, would be useful for making a hybrid GalT enzyme as used in the present invention.

Expression constructs for the GalT and FUT9 enzymes or hybrid enzymes can be made as is well known to a person skilled in the art.

A regulatory DNA sequence is said to be "operably linked to" or "associated with" a nucleotide sequence that codes for a protein if the two sequences are situated such that the regulatory DNA sequence affects expression of the coding nucleotide sequence.

"Regulatory elements" refer to sequences involved in conferring the expression of a nucleotide sequence. Regulator elements comprise a promoter operably linked to the nucleotide sequence of interest and, optionally, 3' sequences, such as 3' regulatory sequences or termination signals. They also typically encompass sequences required for proper translation of the nucleotide sequence.

A "promoter" refers to a DNA sequence that initiates transcription of an associated DNA sequence. The promoter region may also include elements that act as regulators of gene expression such as activators, enhancers, repressors, binding sites for DNA binding proteins, and/or sequences that convey tissue specificity.

A "screenable marker gene" refers to a gene whose expression does not confer a selective advantage to a transformed cell, but whose expression makes the transformed cell phenotypically distinct from untransformed cells.

A "selectable marker gene" refers to a gene whose expression in a plant cell gives the cell a selective advantage. The selective advantage possessed by the cells transformed with the selectable marker gene may be due to their ability to grow in the presence of a negative selective agent, such as an antibiotic or a herbicide, compared to the growth of non-transformed cells. The selective advantage possessed by the transformed cells, compared to non-transformed cells, may also be due to their enhanced or novel capacity to utilize an added compound as a nutrient, growth factor or energy source. Selectable marker gene also refers to a gene or a combination of genes whose expression in a plant cell gives the cell both, a negative and a positive selective advantage.

"Transformation" refers to introduction of a nucleic acid into a cell and in particular, the stable integration of a DNA molecule into the genome of an organism of interest.

Polynucleotide constructs for expression of a gene such as the gene coding for the (hybrid) GalT and FUT9 enzymes in the plant nucleus preferably comprise appropriate 3' sequences, such as 3' regulatory sequences or transcription terminators, to be operably linked downstream of the heterologous nucleotide sequence. Several such terminators are available and known in the art (e. g. tm1 from CaMV, PotPI II from potato, E9 from rbcS). Any available terminator known to function in plants can be used in the context of this invention. Numerous other sequences can be incorporated into polynucleotide constructs for expression of a DNA molecule described in this invention. These include sequences which have been shown to enhance expression such as intron sequences (e. g. from Adh1 and bronzel) and viral leader sequences (e. g. from TMV, MCMV and AMV).

The polynucleotide construct comprises a recombinant polynucleotide for expression of the FUT9 enzyme or hybrid enzyme. Said gene preferably comprises a nucleic acid which codes for a protein according to Fig. 5 or a functional fragment thereof. A functional fragment of said protein is defined as a protein which is highly homologous to the protein depicted in Fig. 5 and which remains functional when expressed in a plant, wherein said functionality means that it is functioning as an α1,3-fucosyltransferase

Highly homologous in this sense means that an amino acid sequence has a sequence identity of more than 50%, preferably more than 70%, more preferably more than 80% and most preferably more than 90% with the above mentioned sequence.

For calculation of percentage identity the BLAST algorithm can be used (Altschul et al., 1997 Nucl. Acids Res. 25:3389-3402) using default parameters or, alternatively, the GAP algorithm (Needleman and Wunsch, 1970 J. Mol. Biol. 48:443-453), using default parameters, which both are included in the Wisconsin Genetics Software Package, Genetics Computer Group (GCG), 575 Science, Madison, Wisconsin, USA. BLAST searches assume that proteins can be modeled as random sequences. However, many real proteins comprise regions of nonrandom sequences which may be homopolymeric tracts, short-period repeats, or regions enriched in one or more amino acids. Such low-complexity regions may be aligned between unrelated proteins even though other regions of the protein are entirely dissimilar. A number of low-complexity filter programs can be employed to reduce such low-complexity alignments. For example, the SEG (Wooten and Federhen, 1993 Comput. Chem. 17:149-163) and XNU (Claverie and States, 1993 Comput. Chem. 17:191-201) low-complexity filters can be employed alone or in combination.

As used herein, 'sequence identity' or 'identity' in the context of two protein sequences (or nucleotide sequences) includes reference to the residues in the two sequences which are the same when aligned for maximum correspondence over a specified comparison window. When percentage of sequence identity is used in reference to proteins it is recognised that residue positions which are not identical often differ by conservative amino acid substitutions, where amino acids are substituted for other amino acid residues with similar chemical properties (e.g. charge or hydrophobicity) and therefore do not change the functional properties of the molecule. Where sequences differ in conservative substitutions, the percentage sequence identity may be adjusted upwards to correct for the conservative nature of the substitutions. Sequences, which differ by such conservative substitutions are said to have 'sequence similarity' or 'similarity'. Means for making these adjustments are well known to persons skilled in the art. Typically this involves scoring a conservative substitution as a partial rather than a full mismatch, thereby increasing the percentage sequence identity. Thus, for example, where an identical amino acid is given a score of 1 and a non-conservative substitution is give a score of zero, a conservative substitution is given a score between 0 and 1. The scoring of conservative substitutions is calculated, e.g. according to the algorithm of Meyers and Miller (Computer Applic. Biol. Sci. 4:11-17, 1988).

Preferably the nucleic acid which codes for the catalytic domain of FUT9 comprises the sequence which codes for the amino acid sequence indicated in bold in Fig. 5. Further preferred, is the nucleic acid sequence coding for the hybrid FUT9 enzyme as depicted in Fig. 4.

The polynucleotide construct of the present invention is preferable constructed such that it comprises at least and in operable linkage a first promoter that is functional in plants, a nucleotide sequence encoding a FUT9 enzyme, and a terminator. Optionally the polynucleotide may comprise a gene sequence encoding a selectable or screenable trait operably linked to regulatory sequences for expression.

Preferably a viral promoter, such as the promoter from cassava vein mosaic virus (CVMV) or the promoter from cauliflower mosaic virus (CMV). However, any promoter that provides for constitutional expression (such as the 35S or the enhanced 35S promoter) may be used. Further, it is a prerequisite that for the formation of Le^{X} epitopes, both the GalT enzyme and the FUT9 enzyme are expressed in the same cells and/or same cell compartment, in which they would also be able to exert their functions to modify the N-glycan of the plant glycoproteins. In this way, also expression of both enzymes regulated by promoters which are tissue or development stage specific would be possible, if only the promoter driving the expression of GalT would have the same expression characteristics as the promoter driving the expression of the FUT9 enzyme. This could be possible by bringing both genes under the control of the same promoter, by using the same promoter twice, i.e. once for each nucleotide sequence . Alternatively, it is possible to provide both the GalT and the FUT9 activities in a fusion protein, wherein at least the catalytic domains of both enzymes are present and functional. Such a fusion protein can then be controlled by the promoter and other regulatory sequences of choice. The fusion protein can also be provided as a hybrid fusion protein, i.e. harboring a sequence coding for an N-terminal domain which confers Golgi localization, such as the N-terminal domain of *Arabidopsis thaliana* xylosyltransferase.

Of course expression of the protein of interest, which should be glycosylated according to the invention, should be regulated in such a way, that the GalT and FUT9 enzymes are able to act upon it. This means that the promoter driving expression of the protein to be glycosylated should be at least active in the tissues or at the times that the promoter(s) driving the expression of the enzymes as described above, are also active.

The recombinant gene constructs may be inserted into a vector, which may be commercially available, suitable for transforming into plants and suitable for expression of the gene product in the transformed cells. Preferably used are binary vectors (such as pMOG22, known from Goddijn, O.J.M. et al., 1993, Plant J, 4:863-873) which are useful for plant transformation using *Agrobacterium.*

In principle any transformation method may be used to introduce chimeric DNA according to the invention into a suitable ancestor cell. Methods may suitably be selected from the calcium/polyethylene glycol method for protoplasts, electroporation of protoplasts, microinjection into plant material, (DNA or RNA-coated) particle bombardment of various plant material, infection with (non-integrative) viruses, in planta *Agrobacterium tumefaciens* mediated gene transfer by infiltration of adult plants or transformation of mature pollen or microspores (EP 0 301 316) and the like. A preferred method according to the invention comprises *Agrobacterium*-mediated DNA transfer. Especially preferred is the use of the so-called binary vector technology as disclosed in EP 0 120 516 and U.S. Patent 4,940,838.

A method for production of a transgenic plant or plant part according to the invention may comprise the step of selecting transformed plants or plant parts. Generally after transformation, plant cells or cell groupings are selected for the transfer with the polynucleotide construct comprising the DNA-sequence with the genes encoding the various enzymes or blocking mechanisms according to the invention, following by steps know to the skilled person by which the transformed material is regenerated into a whole plant and evaluating the transformed plant for the overproduction of glycerol.

Selectable markers, which may be included as a part of the introduced recombinant DNA, are used to select transformed cells (those containing recombinant DNA) over untransformed cells. Examples of suitable markers include genes that provide antibiotic or herbicide resistance. Cells containing the recombinant DNA are capable of surviving in the presence of antibiotic or herbicide concentrations that kill untransformed cells. Examples of selectable marker genes include the bar gene which provides resistance to the herbicide Basta; the nptII gene which confers kanamycin resistance; the hpt gene which confers hygromycin resistance; and the cah gene which gives resistance to cyanamid. An entire plant can be generated from a single transformed plant cell through cell culturing techniques known to those skilled in the art.

A process for obtaining a transgenic plant according to the invention may in an alternative embodiment comprise introducing a vector according to the invention into an ancestor plant, and then producing said transgenic plant from said ancestor plant.

Yet another alternative embodiment for obtaining a transgenic plant according to the invention may comprise introducing a polynucleotide construct according to the invention into a suitable vector for transforming a plant part to produce a transformed plant part, and then regenerating said transgenic plant from said transformed plant part.

Following DNA transfer and regeneration, putatively transformed plants may be evaluated, for instance using Southern analysis, for the presence of the recombinant DNA according to the invention, copy number and/or genomic organization. In addition, or alternatively, expression levels of the newly introduced DNA may be undertaken, using Northern and/or Western analysis, techniques well known to persons having ordinary skill in the art. Further, as is exemplified in the Example, MALDI-TOF MS analysis may be used to analyse the oligosaccharide moieties on the glycoproteins.

Also a part of the invention is the hybrid xylFUT9 enzyme, being a preferred embodiment for providing the fucosyltransferase activity at the desired site. Preferably, the enzyme has the amino acid sequence as provided in Figure 5. It would, of course, be possible to use the human FUT9 catalytic domain or a catalytic domain of a FUT9 enzyme from any other source. Sequences which are highly identical to the pufferfish FUT9 catalytic domain may be constructed by changing the amino acid sequence, and/or the encoding nucleotide sequence. Homologues, which are useful in the present invention are those homologues which have 70% or more, preferably 80% or more, more preferably 90% or more, most preferably 95% or more sequence identity with the catalytic domain identified in the sequence listed below.

In order, as described by Bakker *et al.* (2006) to produce humanized and less allergenic monoclonal antibodies in plants, the protection by the Lewis X epitope should be reversible. In other words, after production and optionally isolation of the antibodies produced in the plant, the fucose moiety, which has been attached to the N-glycan should be removed. This can be performed *in vitro* by an enzyme which removes the fucose moiety, such as an *Xanthomonas manihotis* α1-3,4-fucosidase.

The following enabling Examples serve to further illustrate the invention, and are not intended to define limitations or restrict the scope of the subject invention.

### EXAMPLE 1

### Materials and methods

### Construction plant transformation vectors

A DNA fragment comprising the stem region and catalytic domain of T. *nigroviridis* FUT9a (accession no. AJ783833) was amplified from genomic DNA using the following primers: FUTup, TGACCATGGCGTCTCACATGACTGAATTCTCCTCCGGACCAGTGGAGACA GGACTGA; FUTdw, GTGACGGATCCAATCAACCCCAGTACCACTTGTTAAG. The *Nco* I/Bam HI digested fragment was cloned into a likewise digested pMTL23-derivative lacking the *Eco* 31I site giving clone pMTLtrFUT9 (Chambers *et al.,* 1988). A cDNA fragment covering the N-terminal part of the *Arabidopsis thaliana* xylosyltransferase (accession no. AJ277603) was obtained by amplification with the following primers: AtxylBpi, GTGACGAAGACAACATGAGTAAACGGAATCCGAAGATTC; Atxy1137c, GTGACGAATTCCGA-TTGGTTATTCACTGAAACGT. This fragment was digested with *Bpi* I and *Eco* RI and cloned into *Esp* 3I/*Eco* RI digested pMTLtrFUT9 yielding clone pMTLxylFUT9. The hybrid enzyme was encoded by the DNA sequence from Fig. 4(stop and start codons underlined), providing the amino acid sequence as depicted in Fig. 5.:

The promoter-terminator cassette for cloning of the hybrid gene was made by removing the enhanced 35S promoter from pUCAP35S (van Engelen, F.A., Molthoff, J.W., Conner, A.J., Nap, J-P., Pereira, A. and Stiekema, W.J. (1995) pBINPLUS: An improved plant transformation vector based on pBIN19. Transgenic Res., 4, 288-290.) by *Asc* I/*Nco* I digestion and replacing it with likewise digested PCR fragment covering the cassava vein mosaic virus (CVMV) promoter that had been obtained following amplification with primers: CVMV-Asc, GTCAGGCGCGCCGCGATCGCCAGAAGGTAATTATCCAAG; and CVMV-Nco, GTGACCATGGAACAAACTTACAAATTTCTC-TGAAG (Verdaguer, B., de Kochko, A., Beachy, R.N. and Fauquet, C.M. (1996) Isolation and expression in transgenic plants of the cassava vein mosaic virus (CVMV) promoter. Plant Mol. Biol. 31, 1129-1139.). The *Esp* 3I/*Bam* HI fragment containing the hybrid gene was cloned into *Nco* I/*Bgl* II digested pUCAP-CVMV. This clone was used to insert the xylGalT expression cassette constructed earlier (Bakker *et al.,* 2006). The *Asc*I/*Pac*I fragment comprising the two expression cassettes in the same transcriptional orientation was cloned into a modified version of binary vector pMOG22 in which the *Eco*RI and HindIII sites of the multiple cloning site had been replaced by *Pac*I and *Asc*I*,* respectively (Goddijn *et al.,* 1993). PCR amplifications were carried out using AccuPrime Pfx DNA polymerase (Invitrogen) and the sequences of all PCR fragments were verified using DYEnamic^{™} ET Terminator Cycle Sequencing Kits (GE Healthcare).

### Plant transformation and growth

The plant transformation vector comprising the two genes was used to transform *Nicotiana tabacum* (cv Samsun NN) as described previously (Bakker *et al.,* 2001). Transgenic seedlings and plants were grown in a greenhouse at 21°C.

### N-glycan analysis

N-glycans were purified as described (Bakker *et al.,* 2006). For galactosidase treatments, N-glycans were incubated over night with 1.5 mU *Streptococcus pneumoniae* β1,4-galactosidase (Calbiochem) in 50 mM sodium phosphate buffer pH 6.0 and for fucosidase treatments N-glycans were incubated over night with 0.5 mU *Xanthomonas manihotis* α1-3,4 fucosidase (Sigma-Aldrich) in 50 mM sodium phosphate pH 5.0. Oligosaccharides were purified away from salts and enzymes using a Ultra-Clean Carbograph column as described above. Purified N-glycans were dissolved in 5 mM NaAc and mixed with an equal volume of 1% DHB in 50% acetonitrile. One µl aliquots were spotted onto a stainless steel sample plate and dried under a stream of air at room temperature. Positive-ion MALDI-TOF spectra of [M+Na]⁺ adducts were recorded on a Bruker Ultraflex fitted with delayed extraction, and a nitrogen laser (337nm). A maltodextrin series was used as an external molecular weight standard. Spectra were generated from the sum of 200-300 laser pulses.

### Results

### Construction of plant transformation vectors

The gene fragment encoding the catalytic domain comprising residues 38-359 of a T. *nigroviridis* FUT9a homologue was obtained by PCR using genomic DNA and primers based on accession AJ783833. It was fused N-terminally with the sequence encoding the first 53 aa of the *A. thaliana* XylT gene. Sequencing of a number of independent clones containing the truncated FUT9 gene revealed two non-silent mutations in all of them which lead to aa changes at positions 65 (I to V) and 176 (A to T). Neither of these mutations concerned residues that were highly conserved compared to other animal homologues. The hybrid gene was placed under control of the Cassava Vein Mosaic Virus (CVMV) promoter and the resulting expression cassette was combined with the one comprising the hybrid GalT under control of the CaMV 35S promoter (Verdaguer et al., 1996, Plant Mol. Biol. 31:1129-1139; Bakker *et al.,* 2006). Together they were transferred to a plant transformation vector conferring hygromycin resistance, which was designated xFxG.

### N-glycan profiles from transgenic population

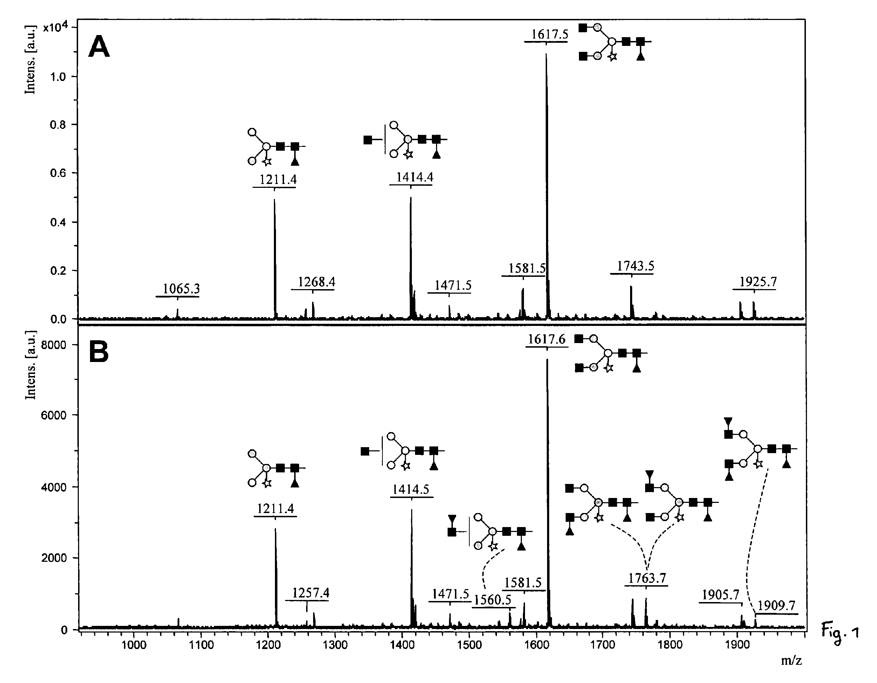

From a group of eleven transgenic plants obtained following transformation with xFxG and growing in the greenhouse, plants were analyzed for N-glycan composition using MALDI-TOF MS. Two transgenics contained the three abundant complex N-glycans that are typical of wild-type plants, whereas five others displayed N-glycan profiles that at first sight appeared to be wild-type as well, but which proved to be supplemented with additional oligosaccharides. For example in Fig. 1, a comparison of the N-glycan profiles from lines xFxG3 and xFxG4 showed clearly that line xFxG4 contained unusual N-glycans at m/z 1560.5, 1763.7 and 1909.7 compared to the typical wild-type N-glycans found in line xFxG3. In other words, they seemed to represent mono- or bifucosylated versions of the typical wild-type products at *m*/*z* 1414.4 and 1617.6.

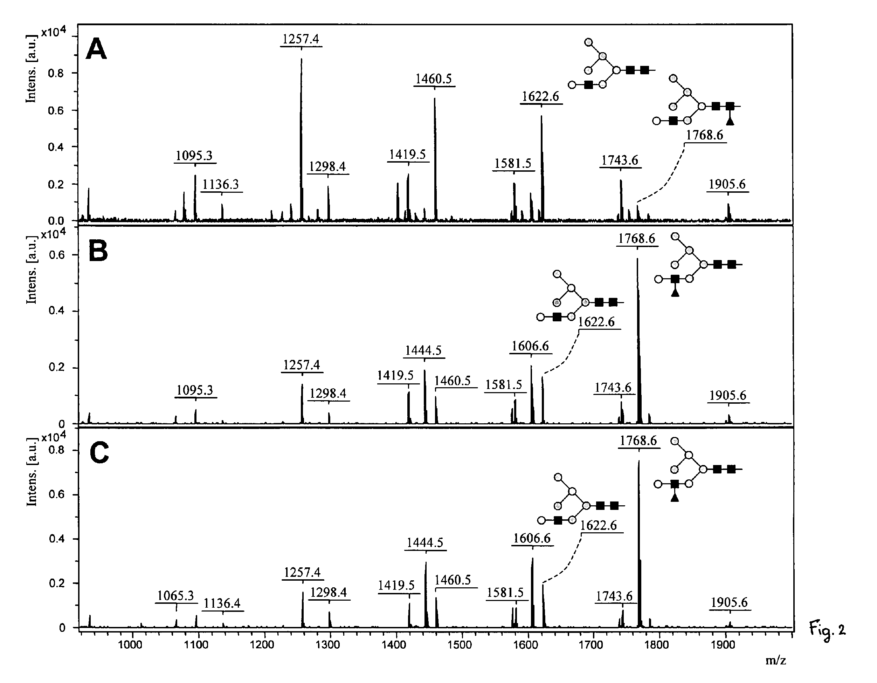

Transgenic line xFxG1 contained N-glycans indicative of the presence of Le^{x} epitopes. A comparison of its N-glycans with that from line xylGalT12 containing only the xylGalT gene revealed clear differences that suggested addition of a fucose residue to the hybrid galactosylated N-glycans that are typical of xylGalT lines (Fig. 2A,B) (Bakker *et al.,* 2006). For example, efficient fucosylation of the most abundant galactosylated N-glycan in xylGalT12 in Fig. 2A at *m*/*z* 1622.6 - GalGlcNAcMan₅GlcNAc₂ - would explain the occurrence of a most abundant hybrid in Fig. 2B at m/z 1768.6. The fact, that PNGase F (an enzyme which is incapable to splice N-glycans with a core bound α1,3-fucose from the protein backbone)-mediated release of the N-glycans gave an N-glycan profile that was virtually indistinguishable from the one obtained following PNGase A(an enzyme which is capable of splicing N-glycans with a core bound α1,3-fucose)-mediated release, showed unequivocally that most of the N-glycans in line xFxG1 - including the peak at m/z 1768.6 in line xFxG1 - lacked the core-bound α1,3-fucose (Fig. 2C). At 20% of total peak area the level of high-mannose is this line is somewhat lower than what is usually encountered in plants expressing only xylGalT.

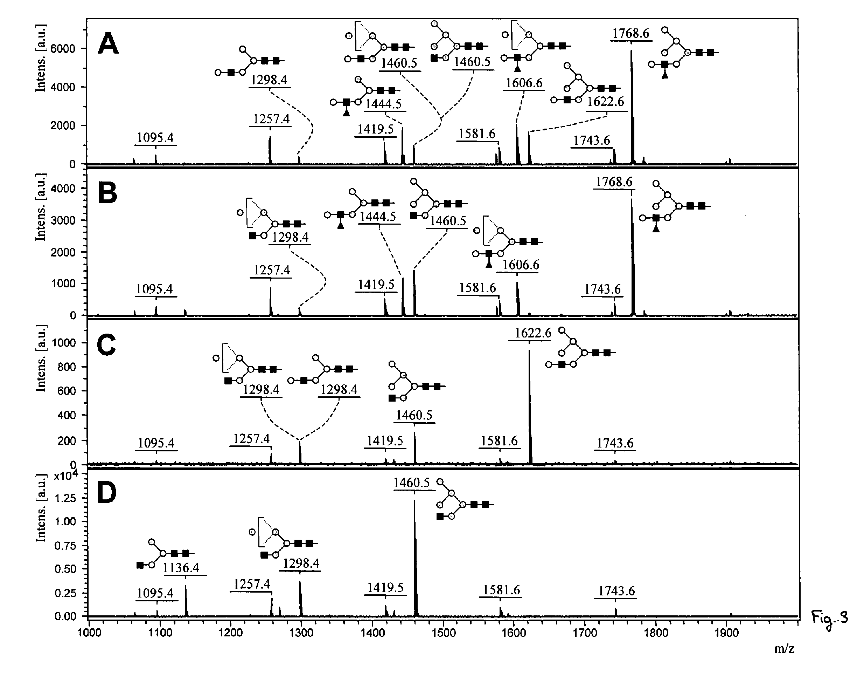

The presence of Le^{x} epitopes in line xFxG1 was corroborated further by sequential treatment of its N-glycans with ß1,4-galactosidase and α1-3,4 fucosidase followed by MALDI-TOF MS analysis. MALDI-TOF MS analysis of ß1,4-galactosidase treated N-glycans from line xFxG1 showed that the products at m/z 1444.5, 1606.6 and 1768.6 were not cleaved (cf. Figs. 3A and 3B), whereas the disappearance of the product at *m*/z 1622.6 proved that the galactosidases had been active. In view of the fact that a plant-specific fucosidase has been identified that could cleave the fucose from Le^{X} epitopes, we can not rule out that the GalGlcNAcMan₅GlcNAc₂ peak at m /z 1622.6 (Fig. 3A) is due to fucosidase-mediated cleavage of the Gal(Fuc)GlcNAcMan₅GlcNAc₂ peak at m/z 1768.6 instead of incomplete conversion of Gal(ß1-4)GlcNAc termini (Zeleny *et al*., 2006).

At the same time, the peak at m/z 1460.5 in this figure was increased, which can be deduced by comparing them with the neighboring high-mannose peaks at m/z 1419.5 and 1581.6, respectively. The extent of the increase of the peak at *m*/*z* 1460.5 in Fig. 3B equals the size of the peak at *m*/*z* 1622.6 in Fig. 3A, which renders it very likely that this increase is due to de-galactosylation of the N-glycan at *m*/*z* 1622.6. Digestion of the β1,4-galactosidase treated N-glycans with α1-3,4-fucosidase revealed not only that the oligosaccharides at *m*/*z* 1444.5, 1606.6 and 1768.6 in Fig. 3B contained fucose residues at their nonreducing ends, but their almost complete cleavage also demonstrated that in fact all three peaks were practically lacking N-glycans with a core-bound fucose (Fig. 3C). Subsequent digestion of the defucosylated products with ß1,4-galactosidase demonstrated the presence of ß1,4-linked galactose residues on the antennae of products at m/z 1298.4 and 1622.6 (Fig. 3C). This is easy to see for the ion at m/z 1622.6 that disappears completely in Fig. 3D. The presence of the galactose residue on the N-glycan at m/z 1298.4 in Fig. 3C can be inferred from the emergence of a peak at m/z 1136.4 representing GlcNAcMan3GlcNAc2 in Fig. 3D upon digestion with ß-galactosidase. To sum up, the observation that ß1,4-galactosidase could only cleave the galactose from the hybrid N-glycans at m/z 1444.4, 1606.5, and 1768.6 in Fig. 3A after removal of the fucose residue demonstrated that they carried Le^{X} epitopes.

## Claims

1. Method to produce a Lewis x epitope on N-linked glycans on glycoproteins in a plant, plant part or plant cells, comprising
d. expression of a nucleotide sequence encoding a galactosyltransferase, which catalyzes the addition of galactose in ß1,4-linkage to N-linked glycans, and
e. expression of a nucleotide sequence encoding a fucosyltransferase, which catalyzes the addition of fucose in α1,3-linkage to the N-acetylglucosamine of Galß1-4GlcNAc structures on N-linked glycans, and
f. expression of one or more nucleotide sequences encoding one or more glycoproteins, which are able to be provided with an N-linked glycan, in said plant, plant part or plant cell

2. Method according to claim 1, wherein said nucleotide sequence encoding a galactosyltransferase is a nucleotide sequence encoding at least the catalytically active domain of human GalT.

3. Method according to claim 2, wherein said nucleotide sequence encoding a galactosyltransferase also comprises an N-terminal domain, which confers localisation to the Golgi apparatus, preferably wherein said N-terminal domain is the N-terminal domain of *Arabidopsis thaliana* xylosyltransferase.

4. Method according to any of claims 1-3, wherein said nucleotide sequence encoding a fucosyltransferase is a nucleotide sequence encoding at least the catalytically active domain of α1,3-fucosyltransferase, preferably FUT9, more preferably the FUT9 enzyme of *Tetraodon nigroviridis..*

5. Method according to claim 4, wherein said nucleotide sequence encoding a fucosyltransferase also comprises an N-terminal domain, which confers localisation to the Golgi apparatus, preferably wherein said N-terminal domain is the N-terminal domain of *Arabidopsis thaliana* xylosyltransferase.

6. Method to render a galactose residue on N-linked glycans in plant glycoproteins insensitive to galactosidase by incorporating it within a Lewis X epitope according to a method according to any of claims 1-5.

7. A hybrid α1,3-fucosyltransferase, comprising an N-terminal domain, which confers localisation to the Golgi apparatus, preferably wherein said N-terminal domain is the N-terminal domain of *Arabidopsis thaliana* xylosyltransferase, and the catalytic domain of a FUT9, preferably *T. nigroviridis* FUT9, said hybrid α1,3-fucosyltransferase preferably having the amino acid sequence from Fig. 5

8. A transgenic plant comprising a nucleotide sequence encoding a ß1,4-galactosyltransferase and a nucleotide sequence encoding an α1,3-fucosyltransferase (FUT9).

9. A transgenic plant according to claim 8, capable of producing Lewis X epitopes on N-linked glycans of glycoproteins.


SEQUENCE LISTING

<110> Plant Research International B.V.

<120> Synthesis of Lewis X epitopes on proteins in plants

<130> P77613EP00

<140> EP 06076248.1
<141> 2006-06-16

<160> 8

<170> PatentIn version 3.3

<210> 1
<211> 57
<212> DNA
<213> Artificial

<220>
<223> Primer FUTup

<400> 1
tgaccatggc gtctcacatg actgaattct cctccggacc agtggagaca ggactga 57

<210> 2
<211> 37
<212> DNA
<213> Artificial

<220>
<223> Primer FUTdw

<400> 2
gtgacggatc caatcaaccc cagtaccact tgttaag 37

<210> 3
<211> 39
<212> DNA
<213> Artificial

<220>
<223> Primer AtxylBpi

<400> 3
gtgacgaaga caacatgagt aaacggaatc cgaagattc 39

<210> 4
<211> 34
<212> DNA
<213> Artificial

<220>
<223> Primer Atxyl137c

<400> 4
gtgacgaatt ccgattggtt attcactgaa acgt 34

<210>  5
<211>  39
<212>  DNA
<213>  Artificial

<220>
<223>  Primer CVMV-Asc

<400>  5
gtcaggcgcg ccgcgatcgc cagaaggtaa ttatccaag                              39


<210>  6
<211>  35
<212>  DNA
<213>  Artificial

<220>
<223>  Primer CVMV-Nco

<400>  6
gtgaccatgg aacaaactta caaatttctc tgaag                                  35


<210>  7
<211>  1131
<212>  DNA
<213>  Artificial

<220>
<223>  Hybrid xylFUT9 enzyme


<220>
<221>  CDS
<222>  (1)..(1131)

<220>
<221>  C_region
<222>  (1)..(159)
<223>  derived from A. thaliana xylosyltransferase

<220>
<221>  N_region
<222>  (160)..(1131)
<223>  derived from T. nigroviridis FUT9

<400>  7
atg agt aaa cgg aat ccg aag att ctg aag att ttt ctg tat atg tta       48
Met Ser Lys Arg Asn Pro Lys Ile Leu Lys Ile Phe Leu Tyr Met Leu
1               5                   10                  15

ctt ctc aac tct ctc ttt ctc atc atc tac ttc gtt ttt cac tca tcg       96
Leu Leu Asn Ser Leu Phe Leu Ile Ile Tyr Phe Val Phe His Ser Ser
            20                  25                  30

tcg ttt tca ccg gag cag tca cag cct cct cat ata tac cac gtt tca      144
Ser Phe Ser Pro Glu Gln Ser Gln Pro Pro His Ile Tyr His Val Ser
        35                  40                  45

gtg aat aac caa tcg gaa ttc tcc gga cca gtg gag aca gga ctg agc     192
Val Asn Asn Gln Ser Glu Phe Ser Gly Pro Val Glu Thr Gly Leu Ser
    50                  55                  60

tcg gag cat gtc aag aat caa cta tca acc aag agt gat aaa aac atg     240
Ser Glu His Val Lys Asn Gln Leu Ser Thr Lys Ser Asp Lys Asn Met
65                  70                  75                  80

acc gtt ata ctg ctg tgg ttc tgg ccg ttc gga caa acc ttc gac ctg     288
Thr Val Ile Leu Leu Trp Phe Trp Pro Phe Gly Gln Thr Phe Asp Leu
                85                  90                  95

aac gtg tgc aac gct tat ttc ggc ttt gag ggc tgt tac att acg gcg     336
Asn Val Cys Asn Ala Tyr Phe Gly Phe Glu Gly Cys Tyr Ile Thr Ala
            100                 105                 110

gac aga aac ctt ttt aac aag tcg gat ggc gtt gtc ttc cac cac aga     384
Asp Arg Asn Leu Phe Asn Lys Ser Asp Gly Val Val Phe His His Arg
        115                 120                 125

gac att gcc ggt gac ctg tcc aac atg ccg aca gtc gtg cga ccg tcc     432
Asp Ile Ala Gly Asp Leu Ser Asn Met Pro Thr Val Val Arg Pro Ser
    130                 135                 140

gtc cag aaa tgg atc tgg ttt aac atg gag tct ccc tca cac tcc tct     480
Val Gln Lys Trp Ile Trp Phe Asn Met Glu Ser Pro Ser His Ser Ser
145                 150                 155                 160

agg cac tct gga atg gag agc ctg ttc aac ctg acg ctg aac tat cgt     528
Arg His Ser Gly Met Glu Ser Leu Phe Asn Leu Thr Leu Asn Tyr Arg
                165                 170                 175

cta gat tct gac att cca ctg cct tat tta tcc gtt gtt gcc act gat     576
Leu Asp Ser Asp Ile Pro Leu Pro Tyr Leu Ser Val Val Ala Thr Asp
            180                 185                 190

act gag gag gac ttt gtc cca cct agc aaa aac aaa ctg gtt tgt tgg     624
Thr Glu Glu Asp Phe Val Pro Pro Ser Lys Asn Lys Leu Val Cys Trp
        195                 200                 205

att gtc agc aac tgg aac cag gaa cat gca cga gtt aaa ttc tac aac     672
Ile Val Ser Asn Trp Asn Gln Glu His Ala Arg Val Lys Phe Tyr Asn
    210                 215                 220

gat ttg tac aag cat gtt gag gtc cac acg tat gga cga gca ttc ggg     720
Asp Leu Tyr Lys His Val Glu Val His Thr Tyr Gly Arg Ala Phe Gly
225                 230                 235                 240

caa cac ttc cca gat gaa gag acc tta cca acc atc gcc agc tgc aag     768
Gln His Phe Pro Asp Glu Glu Thr Leu Pro Thr Ile Ala Ser Cys Lys
                245                 250                 255

ttc tac ctg tca ttt gag aac tcc atc cac aaa gac tac ttt act gaa     816
Phe Tyr Leu Ser Phe Glu Asn Ser Ile His Lys Asp Tyr Phe Thr Glu
            260                 265                 270

aaa ctg tac aac cca ctt gct gtg ggg aca gtg cca gtg gtt ctg ggc     864
Lys Leu Tyr Asn Pro Leu Ala Val Gly Thr Val Pro Val Val Leu Gly
        275                 280                 285

cca cct cgg gag aac tac gag aac atc atc cac gga gat gcc ttt atc     912
Pro Pro Arg Glu Asn Tyr Glu Asn Ile Ile His Gly Asp Ala Phe Ile
    290                 295                 300

cac gtg gaa gac ttt gcc tca gcc aag gag ctt gct gac tat tta ctg     960
His Val Glu Asp Phe Ala Ser Ala Lys Glu Leu Ala Asp Tyr Leu Leu
305                 310                 315                 320

ctc ctg gac aaa aat gag gaa atg tac ctt aaa tat ttt cag tgg cga     1008
Leu Leu Asp Lys Asn Glu Glu Met Tyr Leu Lys Tyr Phe Gln Trp Arg
                325                 330                 335

aga cac ttt aaa gca agg gca gtg gca ttg tgg cct gag gcc acc tgt     1056
Arg His Phe Lys Ala Arg Ala Val Ala Leu Trp Pro Glu Ala Thr Cys
            340                 345                 350

cag gct tgt gat tac gtc agg agg cat aag caa tac aga gta ttc agt     1104
Gln Ala Cys Asp Tyr Val Arg Arg His Lys Gln Tyr Arg Val Phe Ser
        355                 360                 365

gac ctt aac aag tgg tac tgg ggt tga                                 1131
Asp Leu Asn Lys Trp Tyr Trp Gly
    370                 375

<210>  8
<211>  376
<212>  PRT
<213>  Artificial

<220>
<223>  Synthetic Construct

<400>  8

Met Ser Lys Arg Asn Pro Lys Ile Leu Lys Ile Phe Leu Tyr Met Leu
1               5                   10                  15

Leu Leu Asn Ser Leu Phe Leu Ile Ile Tyr Phe Val Phe His Ser Ser
            20                  25                  30

Ser Phe Ser Pro Glu Gln Ser Gln Pro Pro His Ile Tyr His Val Ser
        35                  40                  45

Val Asn Asn Gln Ser Glu Phe Ser Gly Pro Val Glu Thr Gly Leu Ser
    50                  55                  60

Ser Glu His Val Lys Asn Gln Leu Ser Thr Lys Ser Asp Lys Asn Met
65                  70                  75                  80

Thr Val Ile Leu Leu Trp Phe Trp Pro Phe Gly Gln Thr Phe Asp Leu
                85                  90                  95

Asn Val Cys Asn Ala Tyr Phe Gly Phe Glu Gly Cys Tyr Ile Thr Ala
            100                 105                 110

Asp Arg Asn Leu Phe Asn Lys Ser Asp Gly Val Val Phe His His Arg
        115                 120                 125

Asp Ile Ala Gly Asp Leu Ser Asn Met Pro Thr Val Val Arg Pro Ser
    130                 135                 140

Val Gln Lys Trp Ile Trp Phe Asn Met Glu Ser Pro Ser His Ser Ser
145                 150                 155                 160

Arg His Ser Gly Met Glu Ser Leu Phe Asn Leu Thr Leu Asn Tyr Arg
                165                 170                 175

Leu Asp Ser Asp Ile Pro Leu Pro Tyr Leu Ser Val Val Ala Thr Asp
            180                 185                 190

Thr Glu Glu Asp Phe Val Pro Pro Ser Lys Asn Lys Leu Val Cys Trp
        195                 200                 205

Ile Val Ser Asn Trp Asn Gln Glu His Ala Arg Val Lys Phe Tyr Asn
    210                 215                 220

Asp Leu Tyr Lys His Val Glu Val His Thr Tyr Gly Arg Ala Phe Gly
225                 230                 235                 240

Gln His Phe Pro Asp Glu Glu Thr Leu Pro Thr Ile Ala Ser Cys Lys
                245                 250                 255

Phe Tyr Leu Ser Phe Glu Asn Ser Ile His Lys Asp Tyr Phe Thr Glu
            260                 265                 270

Lys Leu Tyr Asn Pro Leu Ala Val Gly Thr Val Pro Val Val Leu Gly
        275                 280                 285

Pro Pro Arg Glu Asn Tyr Glu Asn Ile Ile His Gly Asp Ala Phe Ile
    290                 295                 300

His Val Glu Asp Phe Ala Ser Ala Lys Glu Leu Ala Asp Tyr Leu Leu
305                 310                 315                 320

Leu Leu Asp Lys Asn Glu Glu Met Tyr Leu Lys Tyr Phe Gln Trp Arg
                325                 330                 335

Arg His Phe Lys Ala Arg Ala Val Ala Leu Trp Pro Glu Ala Thr Cys
            340                 345                 350

Gln Ala Cys Asp Tyr Val Arg Arg His Lys Gln Tyr Arg Val Phe Ser
        355                 360                 365

Asp Leu Asn Lys Trp Tyr Trp Gly
    370                 375